# EUROPEAN PATENT APPLICATION

(11) **EP 3 269 328 A2**
(43) Date of publication of application: **17.01.2018**
(21) Application number: 17174708.2
(22) Date of filing: 07.06.2017
(51) Int. Cl.: A61F 2/24

(54) **CARDIAC VALVE IMPLANT**

(30) Priority: 08.06.2016 US 201662347224 P
(71) Applicant: Cook Medical Technologies LLC, Bloomington, IN 47404 (US)
(72) Inventor: MARTINEZ, Michelle, Winston Salem, NC 27104 (US); SURTI, Vihar C., Winston-Salem, NC 27104 (US); MCLAWHORN, Tyler E., Winston-Salem, NC 27106 (US); SIGMON, Jr., John C., Winston-Salem, NC 27101 (US); GITTARD, Shaun Davis, Winston-Salem, NC 27106 (US); HRNICEK, Jillian F., Arvada, CO 80004 (US); HARDY, Gregory James, Winston-Salem, NC 27101 (US)
(74) Representative: Garratt, Peter Douglas

(57) **Abstract**

The present disclosure provides unique medical devices for treating prolapsing valves, as well as associated methods of forming and using those devices. The present disclosure provides cardiac valve implants configured to attach to or brace against patient tissue within the atrium.

## Description

### BACKGROUND

The heart receives oxygenated blood from the pulmonary veins into the left atrium. The left atrium is separated from the left ventricle by the mitral valve (also called the left atrioventricular valve, or the bicuspid valve). During ventricular diastole blood passes through the mitral valve into the left ventricle. During ventricular systole the ventricular muscles contract forcing blood through the aortic valve and out of the heart. The mitral valve must withstand the pressures created during ventricular systole to prevent oxygenated blood from traveling back into the left atrium. The Chordae tendineae prevent prolapse of the mitral valve leaflets by pulling on the leaflets of the mitral valve, holding them in a closed position.

In some instances, for example as a result of disease or trauma, the mitral valve is not able to prevent the backflow of blood from the left ventricle into the left atrium during systole. One cause of mitral regurgitation (MR) is increased leaflet motion, otherwise known as leaflet prolapse. The increased leaflet motion can be due to degeneration of the valve tissue, and/or degeneration or rupture of the chordae tendineae. Leaflet prolapse prevents coaptation of the anterior and posterior leaflet by allowing one or more of the leaflets to extend above the annular plane. A need therefore exists for systems and methods for treating mitral prolapse.

### SUMMARY

In certain aspects the present disclosure provides unique medical devices that can effectively correct mitral valve prolapse without requiring surgical repair or replacement of the anatomical valve. In accordance with some forms of the invention such medical devices are configured to extend between the leaflets of a valve to provide a leaflet contact surface. Accordingly, in one embodiment the present disclosure provides a cardiac valve implant for preventing leaflet regurgitation in a mitral valve, the implant comprising: a leaflet contact member and a brace member. In certain embodiments the leaflet contact member comprises a frame member having a curved portion that is configured to extend into a ventricle upon implantation and is engageable with at least one of the valve leaflets. In some forms the brace member is attached to the leaflet contact member and is configured to extend into the atrium and contact patient tissue, thus securing the leaflet contact member between the leaflets within the valve. In accordance with certain inventive variants the cardiac valve implant further comprises a compliant material secured to the frame member forming a leaflet contact surface which is configured to be engageable with one or more leaflets.

In another embodiment, the disclosure provides a cardiac valve implant for preventing leaflet prolapse in a mitral valve comprising: a frame member having a curved central portion between a first side portion, and a second side portion, the first side portion having a first attachment region, and the second side portion having a second attachment region. In some forms the first attachment region and the second attachment region are securable to patient tissue. In certain embodiments, the curved central portion is configured to extend into the ventricle and is engageable with at least one of the first leaflet or the second leaflet during ventricular systole. In accordance with certain inventive variants the cardiac valve implant further comprises a compliant material secured to the frame member forming a leaflet contact surface which is configured to be engageable with one or more leaflets.

In another embodiment, the disclosure provides a method for treating a mal-functioning mitral valve. In some forms, the disclosed method comprises implanting a cardiac valve implant between the first leaflet and the second leaflet, the cardiac valve implant comprising a frame member having a curved central portion. In some forms the cardiac valve implant has one or more attachment regions for securement to patient tissue. In some forms the cardiac valve implant has one or more brace members configured to extend into the atrium and contact patient tissue to secure the valve implant between the valve leaflets.

In another embodiment, the disclosure provides a method of forming a cardiac valve implant, the method comprising attaching a compliant material to a frame member, wherein the frame member comprises a generally linear shape-memory material and wherein attachment of the compliant material causes the formation of a curve within the frame member.

Additional embodiments as well as features and advantages of embodiments of the invention, will be apparent from the description herein.

### BRIEF DESCRIPTION OF THE DRAWINGS

Fig. 1a is a cross-sectional view of the left side of a human heart.
Fig. 1b is a cross-sectional view of the left side of a human heart having a prolapsing mitral valve.
Fig. 2 is a top-down view of a mitral valve.
Fig. 3 shows one embodiment of a cardiac valve implant according to the present disclosure.
Fig. 4 shows one embodiment of a cardiac valve implant according to the present disclosure implanted within a valve.
Fig. 5a depicts another embodiment of a cardiac valve implant according to the present disclosure.
Fig. 5b depicts another embodiment of a cardiac valve implant according to the present disclosure.
Fig. 5c depicts another embodiment of a cardiac valve implant according to the present disclosure.
Fig. 6 depicts a top-down view of a cardiac valve implant according to the present disclosure.
Fig. 7 illustrates a cardiac valve implant according to the present disclosure loaded within a cannulated delivery device.
Figs. 8a-8b illustrate a delivery catheter introduced into the left atrium of a human heart for delivery of a cardiac valve implant according to the present disclosure.
Fig. 9 is a perspective view of one embodiment of a cardiac valve implant according to the present disclosure.
Fig. 10 is a perspective view of one embodiment of a cardiac valve implant according to the present disclosure.
Fig. 11 is a perspective view of one embodiment of a cardiac valve implant according to the present disclosure.

### DESCRIPTION OF THE SELECTED EMBODIMENTS

For the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates. Though some embodiments of the invention are shown in great detail, it will be apparent to those skilled in the relevant art that some features that are not relevant to the present invention may not be shown for the sake of clarity. Additionally it is to be understood that the drawings are schematic and are not to scale. Instead they are meant to facilitate the understanding of the disclosed invention.

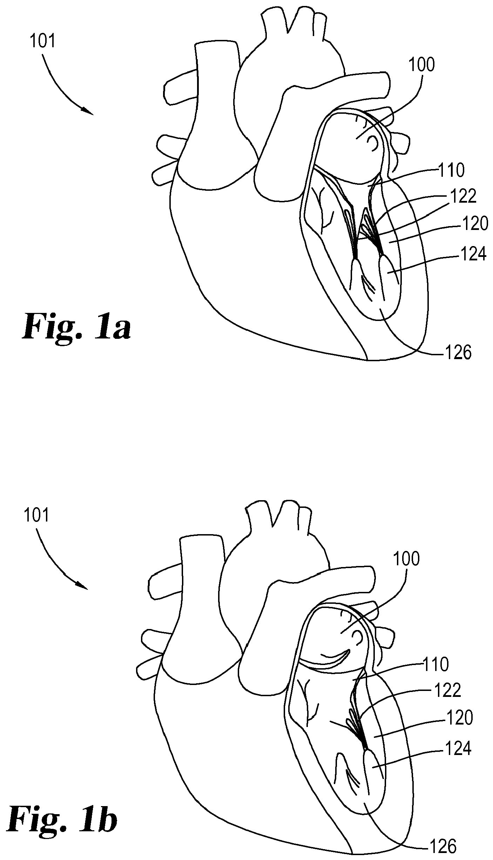

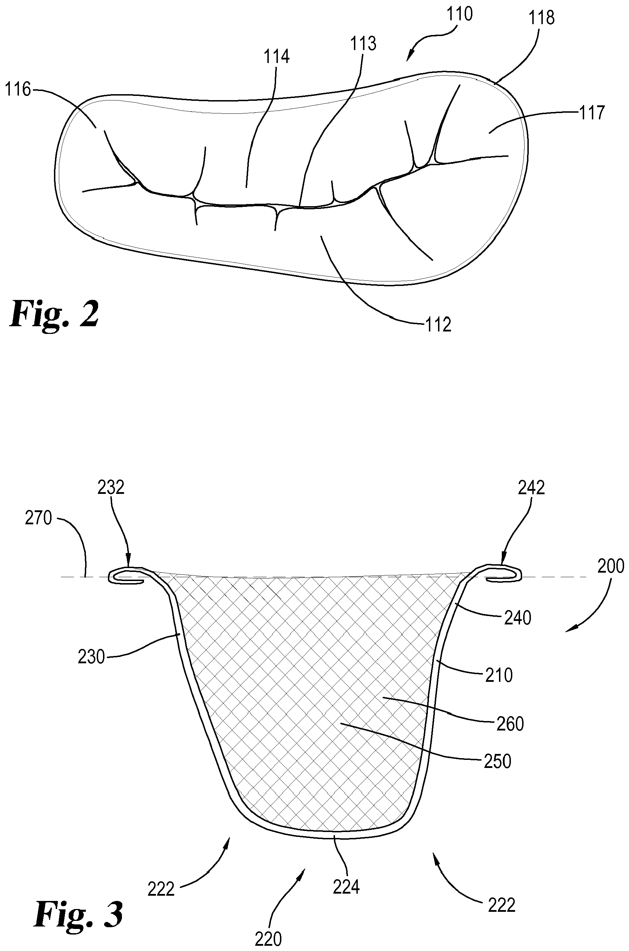

Referring to FIG. 1a, there is shown a cross sectional view of the left side of a human heart 101, including the left atrium 100 and left ventricle 120. The mitral valve 110 is between these two heart chambers and is supported by the chordae tendineae 122. The chordae tendineae extend between each of the anterior and posterior leaflets of the mitral valve to the papillary muscles 124 located near the ventricular apex 126. FIG. 1b depicts one example of a prolapsing mitral valve in which one or more leaflets no longer correctly seals during systole and blood is able to flow back into the atrium. In more seriously damaged valves one or more leaflets enters the atria as depicted in FIG. 1b. With respect to FIG. 2, FIG. 2 shows a top-down view of a healthy mitral valve 110. The anterior leaflet 114, can coapt fully with the posterior leaflet 112, the area where the leaflets meet is the coaptation region 113 which extends between the right (anterior) commissure 116 and the left (posterior) commissure 117. The mitral valve 110 is surrounded by the mitral annulus 118, a fibrous ring that surrounds the valve.

In certain embodiments, the present disclosure provides a device which may be placed within the mitral valve, for example between the anterior leaflet and the posterior leaflet. In some forms, the device provides a surface configured to increase friction between the two leaflets. In some forms, the device provides a surface configured to create sufficient friction between the surface and one or more leaflets such that the damaged leaflet cannot prolapse into the atria, thus preventing mitral regurgitation (MR). In use, the device is placed within the mitral valve with a central portion of the device extending into the left ventricle; this portion provides a surface for contact with the anterior leaflet and/or the posterior leaflet. In some forms, the device is anchored within the atrium with a portion extending into the ventricle.

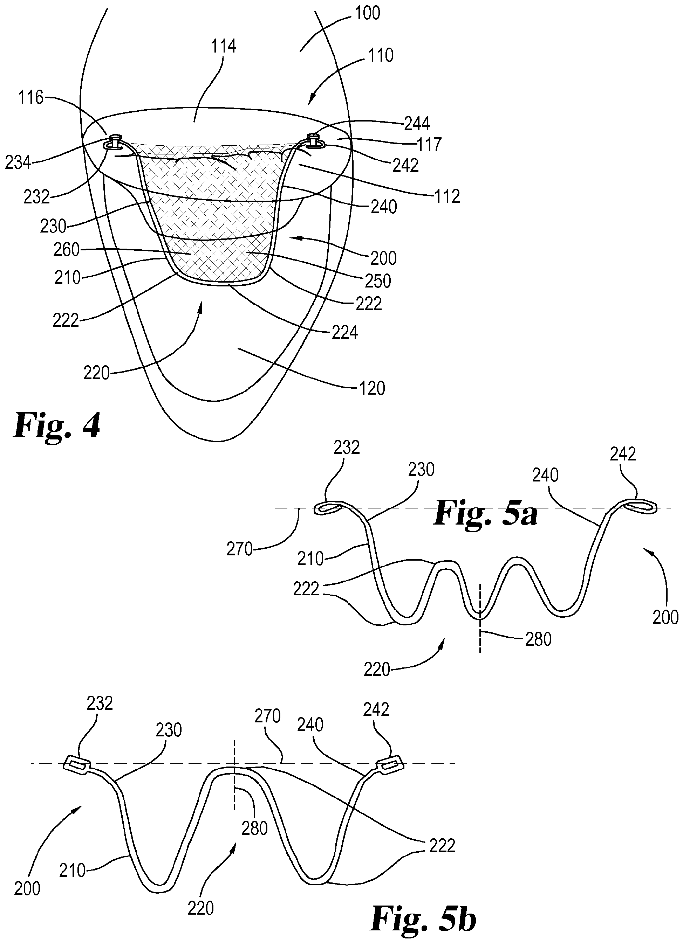

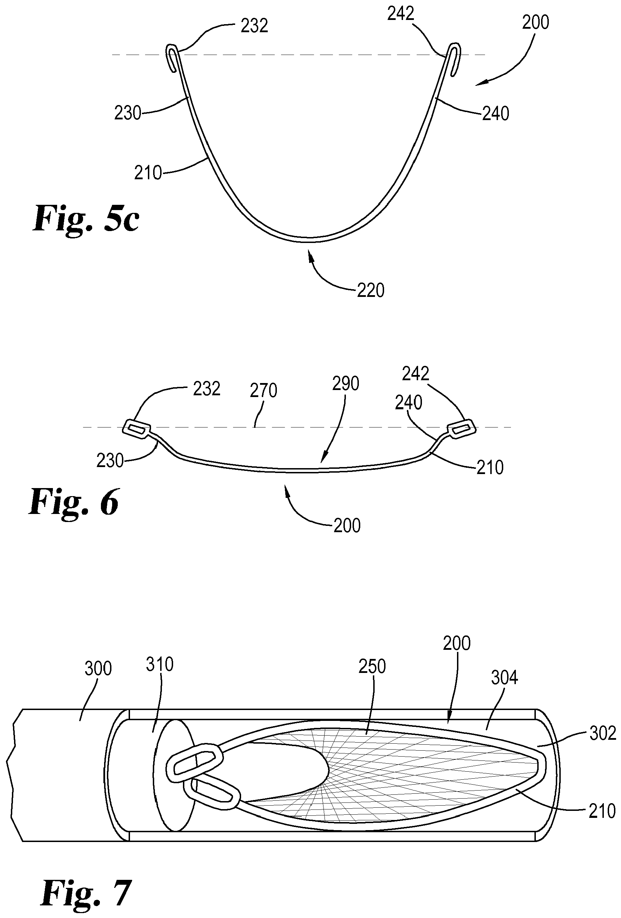

In some modes of practicing the invention, the cardiac valve implant comprises a frame member. In certain embodiments, the frame member comprises a central portion between a first side portion and a second side portion. In some forms, the central portion of the cardiac valve implant is curved forming a curvilinear structure such that upon implantation the central portion is configured to extend into the ventricle. In certain embodiments the central portion may comprise a single curve such as the embodiment shown in figure 5C, however it is also envisioned that the central portion comprise two or more curves defining a serpentine shape as shown for example in figures 5A and 5B. In some modes the central portion comprises a first curve and a second curve with a relatively straight portion between the first and second curves an exemplary embodiment of such a design is shown in figures 3 and 4. In some forms the device is configured such that the apex of the central portion is positioned at the midpoint between the first and second attachment regions such that the device is symmetrical about a vertical axis at the midpoint. It is also envisioned that the device may be asymmetrical about a vertical axis at the midpoint between the first attachment region and the second attachment region. In certain embodiments the frame member has a partially open or concave configuration such that the frame member does not form a closed loop.

In certain beneficial embodiments the frame member is configured to contact the valve leaflet(s) at or near the leaflet edge or periphery. Such a configuration allows for improved coaptation and diminishes stress on the leaflet. It is also envisioned that the frame may be configured to contact the edge of the leaflet at a desired point and a more central portion of the leaflet at another point.

In certain embodiments, the frame member comprises a single piece of material which has been formed into a desired shape. However, it is envisioned that a suitable frame member may be formed from multiple subunits. The frame member may comprise a variety of materials and various percentages and/or combinations of materials. For example, the frame member may comprise a shape memory material such as a nickel titanium alloy such as nitinol. In some forms, the frame member may be compressed into a first condition for loading into a delivery device and is expandable to a second condition suitable for implantation. Other suitable materials for use in constructing the frame member include titanium, stainless steel, tungsten, nitinol, and/or a stiff polymer although any material known to one skilled in the art to be suitable for medical implantation may be used. In some forms, the frame member may be coated with material, for example a drug or bioactive component configured to prevent or promote coagulation or endothelialization after implantation or an electrically insulating material. In embodiments comprising a filament, diameters of about 0.004 inches to about 0.050 inches, more preferably about 0.008 inches to about 0.025 inches.

Frame member size can be variable depending on valve size of the intended application. However, as the disclosed cardiac implant has a certain level of flexibility certain sizes may be designed to fit more than one size of valve, and specific sizing to each individual valve may not be required. Regardless of cardiac implant size, the lengths of first and second side sections should enable center portion and/or leaflet contact surface to be in contact with at least one leaflet after the frame member is implanted inside of a valve.

In accordance with certain inventive variants, the frame member of the present disclosure includes a first attachment region and/or a second attachment region. In certain embodiments, the attachment regions are configured to secure the frame member at or near fibrous tissues surrounding the valve opening, such as valve commissures, valve annulus, and/or trigones. In some forms, first attachment region and or second attachment region is(are) configured to attach directly to the commissure(s), for example the attachment regions may include grasping features such as hooks, barbs, or compression members configured to grasp patient tissue at or near the commissure. In some forms, the attachment regions are configured to accept an anchor member or the like in order to secure the frame member within the valve. Exemplary anchor members include but are not limited to: sutures, staples, and/or surgically implanted posts.

In some forms, the attachment regions are engageable with one or more anchor member(s). The anchor member(s) may be implanted prior to, concurrently, or after introduction of the frame member. In certain embodiments, the anchor members are configured for implantation into fibrous tissues surrounding the valve opening, such as valve commissures, valve annulus, and/or trigones, while also providing an engageable surface for attachment of the frame member. In accordance with certain inventive variants, the anchor members may be attached to a suture as will be described herein.

Anchor members for use in the present disclosure may be comprised of a variety of shapes and materials not limited to those illustrated or described below. In some forms, the anchor members may comprise a wire shaped into a helix, or other formation. In certain embodiments, the anchor member comprises a screw. Alternatively the anchor member may comprise a hook or hooks. In certain embodiments, the anchor member(s) comprise(s) one or more sutures. Some variants of the anchor portion(s) may be combined with a cannula for easier implantation. For example, a cannula can be designed to engage with an anchor member to torque or otherwise manipulate the anchor member. Manipulating the cannula can apply sufficient torque and pressure to anchor member to allow it to penetrate patient tissue at the desired location.

In further embodiments, the anchor portion(s) can have at least one region that has a greater width than the opening in the corresponding embodiments of attachment regions. In some aspects, a region with a relatively greater width can help prevent attachment region from dislodging from the valve.

To aid with placement of anchor portions inside of the valve some embodiments of the disclosed device can also comprise one or more receiving sutures. In some forms, a distal end of a suture can be attached to an attachment region, with a proximal end of the suture extending into a deployment catheter. Such a configuration can be utilized to allow anchor portion to be correctly positioned with attachment region. For example, in some forms the suture may be used as a guide for placing one or more anchor members at or near the attachment region(s). In some forms the anchor members may be configured with a suture receiving portion, for example a cannula configured to receive the suture, such that the anchor member can pass over the suture towards the attachment region. In an alternative embodiment the anchor portion(s) and attachment region(s) are not separate prior to implantation. For example the attachment region may comprise a threaded portion already engaged with a corresponding anchor member. In some forms, the anchor portion may contain an adaptation for preventing separation from the attachment region during implantation.

In some forms the attachment regions and/or anchor members are configured to promote tissue growth at the attachment site. For example, in certain embodiments the attachment region may further comprise a coating or section of material intended to promote tissue growth around the attachment region following implantation. The material may include but is not limited to: a metallic mesh, or extracellular matrix material (ECM), for example small intestinal submucosa (SIS). In addition, the material section, the attachment region, and/or the anchor member can a have a rough surface adapted to encourage endothelialization. In some forms the material section, the attachment region, and/or the anchor member may be coated with a bioactive substance, for example a growth factor or drug. The material section may be attached to the device in any suitable manner.

In certain embodiments, the device of the present disclosure comprises a resilient frame member. In some forms the resilient frame member is constrained when implanted within a valve such that the resilient force of the frame member secures the frame member within the valve opening. For example, as described herein the frame member of the present disclosure may be formed of shape-memory materials. In certain embodiments, upon implantation the device of the present disclosure may impart a radial force of between about 0.02 to about 1.00 lbs. The radial force of the frame member should be such that the frame member is secured within the valve opening without causing harm to surrounding tissues.

In accordance with certain inventive variants, the cardiac valve implant of the present disclosure may also include one or more brace members. As described herein a brace member is a portion of the cardiac valve implant configured to extend into the atria upon implantation and prevent dislodgement of the cardiac valve implant. In some forms a brace member is configured to prevent retrograde motion of the implant. For example, in certain embodiments one or more brace members are configured to contact patient tissue within the atrium in order to resist fluid pressure and secure the cardiac valve implant within the valve opening. In some forms the brace members are configured to conform with patient anatomy, for example to avoid entering the pulmonary veins. In certain embodiments, brace members are configured to extend into a pulmonary vein. In certain embodiments one or more brace members are configured to extend as a loop from the frame member forming an opening having a length of about 2mm to about 50mm, preferably about 15mm to about 30mm, and a width of about 2mm to about 30mm, preferably about 4mm to about 20mm.

In some forms, the entirety of the frame member, including any attachment regions and/or brace members, can be formed of single length of wire. In accordance with certain embodiments the attachment region(s) and/or brace member(s) may comprise a different material from the frame member. In other embodiments, the attachment region(s) and/or brace member(s) are formed from the same material as the rest of the frame member. In some forms, the ends of the wire forming the frame member can be shaped so as to form an attachment region and/or a brace member. For example, in some embodiments a first side of the wire can be bent into a circular loop creating a first attachment region and/or first brace member. In some modes, the first attachment region or first brace member can be bent away from the center portion at an angle to the frame member so as to allow for tissue contact at a desired location (e.g. commissure, trigone, annulus, etc.). In some embodiments a second side of the wire can be bent into a circular loop creating a second attachment region and/or second brace member. In some modes the second attachment region or second brace member can be bent away from the center portion at an angle to the frame member so as to allow for tissue contact at a desired location (e.g. commissure, trigone, annulus, etc.). In some forms, the attachment region(s) and/or brace member(s) may comprise, for example a hook, a flattened portion, or an aperture within the frame material. The second attachment region can be formed substantially similar to the first attachment region, or in certain embodiments the second attachment region may differ from the first attachment region. Similarly, additional brace member(s) can be formed substantially similar to a first brace member, or in certain embodiments the additional brace member(s) may differ from the first brace member. For example, in some forms it may be advantageous to bend the first attachment region and/or brace member differently from the second attachment region and/or brace member so as to allow for tissue contact at desired sites and or to avoid pulmonary veins. Additional configurations of the attachment regions will be apparent to one skilled in the art.

In certain embodiments, the attachment regions and/or brace members are offset from the frame member such that the attachment regions and/or brace members are in a different plane from the curvature of the frame member. In certain forms, the first attachment region and/or first brace member can be in alignment with left valve commissure and the second attachment region and/or second brace member can be in alignment with right valve commissure.

In certain inventive variants, the central portion of the frame member provides sufficient surface area so as to coapt with one or more leaflets. Modifications are envisioned to increase the surface area of the frame member available for leaflet contact. Examples of such modifications include: a serpentine central portion, a material covering, and/or varied thickness of frame member.

In some forms, the cardiac valve implant of the present disclosure further comprises a compliant material attached to the frame member. In some forms the compliant material comprises a biocompatible compliant material. In certain embodiments, a compliant material covers at least the central portion of the frame member so as to create a leaflet contact surface on the central portion of the cardiac valve implant. In certain embodiments, this compliant material is substantially two-dimensional. Suitable constructs for forming the leaflet contact surface may comprise some combination or percentage of the following: a mesh, a film, a fabric, a weave, a knit, a matrix, or a non-woven material.

Examples of materials for the compliant material are polyesters, polyethers, polyolefins, polyurethanes, fluoropolymers, polyamides, elastomers, metals, and/or biologics. In preferred embodiments the compliant material is blood-permeable. Additional materials that can be attached to frame member, will be apparent to one skilled in the art.

In accordance with certain inventive variants the compliant material is configured to restrain a resilient frame member. For example, in some forms the frame member comprises a substantially straight wire comprised of a shape memory material as discussed herein. In some forms attachment of the compliant material causes the resilient frame member to bend, creating a degree of resilient tension within the device. In certain embodiments the device is configured so that upon implantation the frame member is further constrained thus creating slack within the compliant material. As used herein the term "slack" refers to amount of constraint placed upon the compliant material. In certain embodiments the compliant material has a starting width when unconstrained, and a constrained width when deployed within a valve. The slack of the compliant material refers to the percentage difference between the starting width and the constrained width of the compliant material, e.g. (starting width-constrained width)/(starting width)%. In some forms the slack of the compliant material is between about 5 to about 50%, in more preferred forms the slack of the compliant material is between about 15% to about 35%.

In certain embodiments, the compliant material comprises a naturally derived material. Suitable compliant materials can be provided by collagenous extracellular matrix (ECM) materials possessing biotropic properties. For example, suitable collagenous materials include ECM materials such as those comprising submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. Collagenous matrices comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source. For additional information as to some of the materials useful in the present invention, and their isolation and treatment, reference can be made, for example, to U.S. Patent Nos. 4,902,508, 5,554,389, 5,993,844, 6,206,931, and 6,099,567.

The compliant material may further comprise a material which is at least partially permeable to blood flow. In some forms, the compliant material is secured to the frame member and forms a planar or luminal leaflet contact surface that is engageable with at least one valve leaflet. In some embodiments, the compliant material can have sufficient flexibility to expand with the movement of the valve leaflets. Alternatively, the compliant material may provide a certain amount slack to allow the anterior leaflet to coapt with the posterior leaflet while also catching the damaged leaflet to prevent leaflet prolapse.

The compliant material may be attached to the frame member in any suitable fashion. In some modes, the compliant material is attached using a biocompatible adhesive or is heat sealed to the frame member. In certain embodiments, the compliant material is sewn to the frame member. It is also envisioned that the compliant material may comprise perforations or holes of sufficient size so as to allow the frame member to pass therethrough. Such materials include meshes and net-type materials. A similar attachment technique includes creating holes in the compliant material so as to allow the frame member to pass therethrough. In a further embodiment the frame member can have a recess that extends at least though the center portion, and can also extend through sections of first side section and/or second side section, the compliant material surface is securable inside of the recess. The recess need not be continuous around frame member, although in some forms the recess is continuous around the frame member.

The compliant material for use with the present disclosure may comprise one or more layers of material. In certain embodiments, a material with multiple layers is configured to form a pocket which can be secured around the frame member. Further, the compliant material may be secured to the frame member using a combination of any of the techniques described herein. Additional forms of attachment not expressly described will be apparent to one skilled in the art. In certain embodiments the compliant material has a height extending from a lowermost edge to an uppermost edge when attached to the frame member. In some forms the height of the compliant material is between about 5mm to about 70mm, preferably between about 15mm to about 60mm, and most preferably between about 25mm to about 55mm. In certain embodiments the compliant material has a width extending from a first lateral edge to a second lateral edge. In some forms the width of the compliant material is between about 5mm to about 70mm, preferably between about 15mm to about 60mm, and most preferably between about 20mm to about 50mm.

Turning now to a discussion of the illustrated embodiments, Figure 3 depicts one embodiment of a cardiac valve implant according to the present disclosure. Cardiac valve implant 200 comprises a frame member 210 having a first side portion 230, a central portion 220, and a second side portion 240. The illustrated embodiment includes a first attachment region 232 and a second attachment region 242. In the illustrated embodiment, central portion 220 includes curved regions 222, and lower portion 224. In the illustrated embodiment, lower portion 224 is relatively parallel to the attachment plane 270, however it is envisioned that the lower portion may adopt other configurations including those resulting from embodiments wherein the first side of the frame member and the second side of the frame member have differing lengths such that the lower portion is angled relative to the attachment plane. It will also be apparent from the discussion herein that it is envisioned that in some forms the central portion may not include a linear portion and may adopt a gradual or sharp curve. In preferred embodiments, the lower portion adopts a curvilinear shape to reduce changes to blood flow around the device and prevent damage to surrounding tissues. In other embodiments, the frame member may comprise multiple lower portions.

The embodiment illustrated in Figure 3 includes compliant material 250 forming leaflet contact surface 260. It will be understood that each face of the compliant material is capable of coaptation with the anterior leaflet and/or the posterior leaflet, either alone or simultaneously.

Figure 4 depicts the cardiac valve implant of Figure 3 implanted within a mitral valve. Mitral valve 110 defines the boundary between left atrium 100 and left ventricle 120 and includes posterior leaflet 112, anterior leaflet 114, anterior commissure 116, and posterior commissure 117. As described above, cardiac valve implant 200 comprises a frame member 210 having a first side portion 230, a central portion 220, and a second side portion 240. The illustrated embodiment includes a first attachment region 232 and a second attachment region 242. In the illustrated embodiment, center portion 220 includes curved regions 222, lower portion 224, and compliant material 250 forming leaflet contact surface 260.

The embodiment illustrated in Figure 4 also includes first anchor member 234 and second anchor member 244. In the illustrated embodiment the anchor members are implanted at or near the commissures. As discussed herein it is envisioned that the anchor members may be implanted at any suitable location, including for example the valve commissures, the trigones, and/or the valve annulus.

Turning now to Figures 5a-5c which depict various examples of embodiments of frame members for use with the cardiac valve implant of the present disclosure. It will be understood that the embodiments illustrated in figures 5a-5c may be further configured with other adaptations described herein (e.g. compliant material, and/or anchor members). Fig. 5a illustrates an example of one embodiment of a frame member for use in a cardiac valve implant. Fig. 5b illustrates another example of one embodiment of a frame member for use in a cardiac valve implant. Fig. 5c illustrates yet another example of one embodiment of a frame member for use in a cardiac valve implant. In embodiments in which the central portion adopts a serpentine shape, for example figs. 5a and 5b, it is envisioned that the centermost portion 280 may be concave with respect to the attachment plane (as in the embodiment illustrated in Fig. 5a) or convex with respect to the attachment plane (as in the embodiment illustrated in Fig. 5b).

In certain embodiments, the frame member has an overall arc shape similar to that of the coaptation region between the anterior and posterior leaflets of the mitral valve. Fig 6 illustrates a top-down view of one embodiment of a cardiac valve implant 200, having frame member 210. In the illustrated embodiment, frame 210 has an arc 290 which conforms to the coaptation region between the anterior and posterior leaflets of the mitral valve.

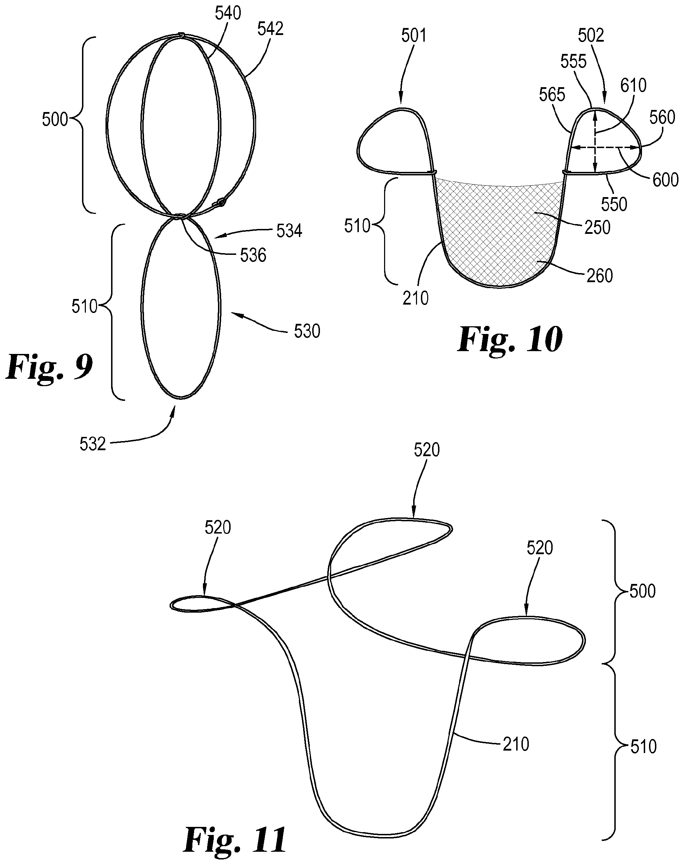

Figure 9 illustrates one embodiment of a cardiac valve implant according to the present disclosure. Cardiac valve implant 200 comprises a brace member 500 and a leaflet contact member 510. In the illustrated embodiment leaflet contact member 510 comprises a leaflet contact hoop member 530 having a lower portion 532 and an upper portion 534. Upper portion 534 of leaflet contact member 510 forms an attachment 536 with brace member 500. In the illustrated embodiment brace member 500 comprises interlocking brace hoops 540 and 542 which are configured to form a generally spherical body. In certain embodiments the brace member is sized and shaped so that upon deployment the brace member resiliently pushes against the walls of the atrium thus securing leaflet contact member within a valve structure. In the illustrated embodiment cardiac valve implant 200 is formed from a continuous length of wire, although other configurations are contemplated as discussed herein.

Figure 10 illustrates another embodiment of a cardiac valve implant according to the present disclosure. The illustrated cardiac valve implant comprises a frame member 210 which forms leaflet contact member 510 and brace members 500, 501. The embodiment illustrated in Figure 10 includes compliant material 250 forming leaflet contact surface 260. It will be understood that each face of the compliant material is capable of coaptation with the anterior leaflet and/or the posterior leaflet, either alone or simultaneously. In certain embodiments frame member 210 is a resilient shape memory wire that adopts a generally linear configuration when relaxed. In some forms compliant material 250 is attached to frame member 210 in such a way so as to cause frame member to adopt a curved configuration as shown.

It is envisioned that cardiac valve implants as presently disclosed be sized and shaped to fit within a cardiac valve between a first and second leaflet. In some forms a cardiac valve implant may have a width, extending from a first side portion to a second side portion that is greater than the intercommisural distance. As used herein the term "intercommisural distance" refers to the linear distance between the left commissure and the right commissure. In certain embodiments the cardiac valve implant width is 5-50% larger than the intercommisural distance. In some embodiments the cardiac valve implant width is 10-30% larger than the intercommisural distance. The width of the cardiac valve implant depends on various factors including the frame material and/or the attachment of a compliant material to constrain the frame member. In some forms the width of the cardiac valve implant may be adjustable in situ, for example by adding slack or tension to the compliant material.

In order to prevent migration of the device into the ventricle during diastole, the ratio of the drag force of blood on the implant to the anchoring force of the implant must be less than 1. This securement is achieved by a balance of the drag force to the radial force of the implant. The radial force is dependent on the shape, thickness, and material of the frame member and/or brace or anchor members. The drag force is dependent on the porosity and slack of the compliant material.

In accordance with certain inventive variants a cardiac valve implant may comprise a plurality of brace members, each configured to contact patient tissue and support the leaflet contact member within the valve. For example the embodiment illustrates in Figure 10 comprises a first brace member 501 and a second brace member 502. In the illustrated embodiment the brace members comprise loops of frame member 210. The loops have a height between a first portion 550 and a second portion 555, and a width between a third portion 560 and a fourth portion 565. In accordance with certain inventive variants the brace member is sized and configured in order to avoid anatomical features such as pulmonary veins. In accordance with certain inventive variants the brace member is sized and configured in order to contact anatomical features such as atrial walls, or to extend into a vessel. In some forms the brace member has a length of between about 2mm to about 50mm, preferably about 15mm to about 30mm, and a width of between about 2mm to about 30mm, preferably about 4mm to about 20mm. While the illustrated embodiment has multiple brace members that form loops, other suitable shapes and configurations are envisioned such as helixes, spheres, and arches. Further the shape, dimensions, and orientation of the first brace member are not necessarily consistent with those of the second brace member.

Figure 11 illustrates another embodiment of a frame member for use as a cardiac valve implant according to the present disclosure. In the illustrated embodiment frame member 210 forms a series of turns 520 forming the brace member 500. In the illustrated embodiment brace member 500 and leaflet contact member 510 are formed of a contiguous frame member 210.

In accordance with certain embodiments the brace member and/or leaflet contact member may comprise different materials. In certain inventive variant the brace member may comprise a frame member having an increased diameter relative to that of the leaflet contact member. For example in certain embodiments the brace member may comprise a material having increased resiliency relative to the material of the leaflet contact member. Such a configuration may be desirable to secure the device within the valve while allowing for increased mobility of the leaflet contact member.

The present disclosure also contemplates methods of treatment of a patient suffering from a mal functioning cardiac valve. In some forms, the method comprises implanting a cardiac valve implant as described herein. In certain embodiments, the cardiac valve implant is delivered surgically. In accordance with certain inventive variants, the cardiac valve implant is compressed to a first state for introduction into a cannulated delivery device (e.g. catheter), and expandable to a second deployed state for insertion into the cardiac valve. In some forms, the cardiac valve implant is self-expanding. In some forms, the cardiac valve implant is manually expanded, for example by using a balloon or forceps. In some forms, the methods of treatment described herein include the step of introducing a cannulated delivery device to the atrium of the target valve. Such introduction can be accomplished for example via transseptal access gaining access to the left atrium via the right atrium, via transapical access, or the left ventricle via the aorta.

Figure 7 illustrates one embodiment of a cardiac valve implant loaded within a cannulated delivery device. In the illustrated embodiment, catheter 300 includes lumen 304 defined by inner surface 302. The illustrated embodiment also includes pushing member 310. In certain embodiments, cardiac valve implant 200 is loaded within the lumen, in the illustrated embodiment cardiac valve implant 200 retains a compressed condition due to contact with inner surface 302. In some forms the cardiac valve implant is held in a compressed condition by a retention member, for example a suture or similar construct. As discussed above, in some forms the cardiac valve implant is manually expanded, in such forms an expansion member, for example a balloon, suture, or forceps, may be introduced via catheter 300 as well.

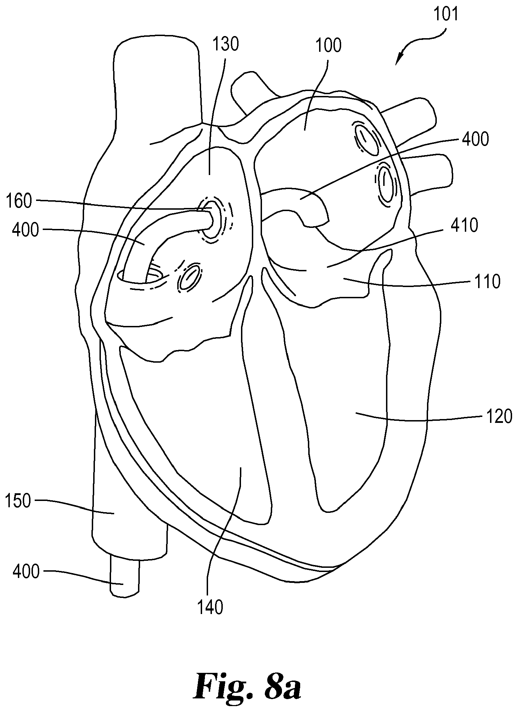

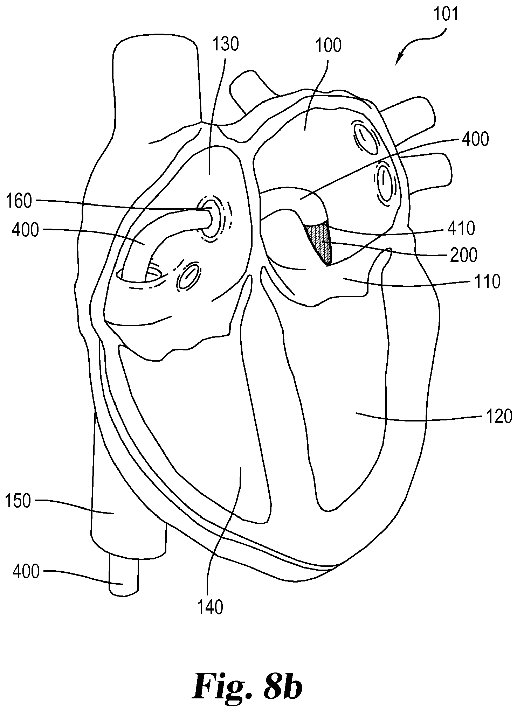

In one method of implantation cardiac valve implant can be compressed for deployment, and put inside of a catheter or other delivery device that can enter the heart. After the delivery device has entered the atrium, for example via transseptal access gaining access to the left atrium via the right atrium, the cardiac valve implant can be deployable near or in the valve annulus. In embodiments in which the cardiac valve is self-expanding the cardiac valve will return to its first uncompressed configuration without any assistance. In embodiments which are not self-expanding the valve implant can be manually expanded using an expansion member such as a balloon or forceps. An operator may further move the implant, such that first attachment region and second attachment region are near or each valve commissure respectively. Certain inventive variant include the step of implanting one or more anchor members prior to or in conjunction with placement of the cardiac valve implant. In some modes, after implantation the cardiac valve implant is anchored within the atrium with the first side section and second side section extending through the valve and into the ventricle. One embodiment of a method of implantation is illustrated in Figures 8a-8b. Figures 8 a and 8b illustrate a delivery catheter 400 inserted into the left atrium 100. In certain embodiments, the delivery catheter is introduced via the left or right femoral vein, passed though the inferior vena cava 150 into the right atrium 130. In the illustrated embodiment the left atrium 100 is accessed via transseptal access through the interarterial septum 160. In some forms, cardiac valve implant 200 is pushed from the distal end 410 of delivery catheter 400 and implanted using the techniques described herein.

In another illustrative embodiment, the frame member is at least partially formed of a non-shape memory metal. Here cardiac implant is compressed to a reduced profile and placed in a catheter or other deployment device. After, gaining access to the left atrium the device is deployed in or near the valve opening. After deployment, the cardiac implant is expanded to a formation that will fit correctly in the valve. Expansion device and techniques include but are not limited to, using forceps, an inflatable object like a balloon, or having sutures that can be manipulated by the operator outside of the body. Other methods of expanding the frame should be apparent to one skilled in the art.

In one exemplary embodiment first attachment region is connected to the first anchor member via a suture, the delivery device is configure to allow an operator to implant the first anchor member at a desired location. In some forms, the second attachment region is also connected to a second anchor portion via a second suture, the delivery device configured to allow an operator to implant the second anchor member at a desired location. With one or more anchor members implanted the cardiac valve implant may be introduced and delivered into position. In some forms the anchor portion(s) is(are) slidably associated with the suture(s) so as to allow the sutures to guide the cardiac valve implant into place within the valve. In a further embodiment a cannula is engaged with said first anchor portion, and a second cannula is engaged with second anchor portion. First cannula and second cannula are connected to first and second sutures respectively such that operator by manipulated suture can torque, and/or apply force to first and second anchor portions.

In certain embodiments, the cardiac valve device may be contained in a sterile packaging prior to use. Sterilization of the packaging may be achieved, for example, by irradiation, ethylene oxide gas, or any other suitable sterilization technique. The device of the present disclosure may also be present as part of a kit which may include the delivery catheter, and/or the expansion member.

### Listing of Certain Embodiments

1. A cardiac valve implant for preventing regurgitation in a mitral valve, the mitral valve having a fibrous annulus and leaflets including a first leaflet, and a second leaflet, the leaflets defining a coaptation region between an atrium and a ventricle, said cardiac valve implant comprising:
   a frame member having a curved central portion between a first side portion, and a second side portion, wherein said first side portion and said second side portion are configured to extend into the atrium, and wherein said curved central portion is configured to extend into the ventricle.
2. The valve implant of embodiment 1, wherein said curved central portion is configured to be engageable with at least one of the first leaflet or the second leaflet during ventricular systole
3. The valve implant of embodiment 1, wherein a compliant material is secured to the frame member forming a leaflet contact surface, said leaflet contact surface configured such that said leaflet contact surface is engageable with one or more valve leaflets.
4. The valve implant of embodiment 3, wherein said compliant material is secured to said frame member at said curved central portion.
5. The valve implant of any one of embodiments 1-4, wherein said frame member comprises a shape memory alloy, and said valve implant has a first state with a reduced profile for implantation, and a second deployed state with a larger width.
6. The valve implant of any one of embodiments 2-5, wherein said compliant material comprises a mesh, a film, a fabric, a weave, a knit, a matrix, or a non-woven material
7. The valve implant of embodiment 1, wherein said first side portion comprises a first loop and said second side portion comprises a second loop.
8. The valve implant of embodiment 1, wherein said first side portion comprises a first attachment region securable to patient tissue, and wherein said second side portion comprises a second attachment region securable to patient tissue.
9. The valve implant of embodiment 7, wherein said first loop and said second loop each have a width between 2mm and 30mm.
10. The valve implant of embodiment 7, wherein said first loop and said second loop each have a height between 2mm and 50mm.
11. The valve implant of embodiment 8, wherein said first attachment region is configured to engage a first anchor member, and said second attachment region is configured to engage a second anchor member.
12. The valve implant of embodiment 1, wherein a section of said frame member comprises a serpentine shape.
13. The valve implant of embodiment 7, wherein said first loop is formed from a first end of said frame member, and wherein said second loop is formed from a second end of said frame member.
14. The valve implant of embodiment 3, wherein said compliant material is blood permeable.
15. The valve implant of embodiment 3, wherein said compliant material has multiple layers.
16. A method for treating a mal-functioning mitral valve, the mitral valve having at least an annulus, a first leaflet, and a second leaflet, defining a coaptation region between an atrium and a ventricle, said method comprising;
   implanting a cardiac valve implant within the coaptation region between the first leaflet and the second leaflet, the cardiac valve implant having a frame member having a curved central portion between a first side portion and a second side portion, and wherein said implant is positioned such that the side portions extend into the atrium and the curved central portion extends into the ventricle and is engageable with at least one of the leaflets during ventricular systole.
17. The method of embodiment 16, further comprising the step of providing a cardiac valve implant within a cannulated delivery device, the cardiac valve implant being expandable from a first compressed state when within the delivery device to a second deployed state when outside of the delivery device.
18. The method of embodiment 17, further comprising the step of introducing the cannulated delivery device into the atrium of the patient.
19. A cardiac valve implant for preventing leaflet regurgitation in a mitral valve, the mitral valve having a fibrous annulus and leaflets including a first leaflet, and a second leaflet, the leaflets defining a coaptation region between an atrium and a ventricle, said cardiac valve implant comprising:
   a leaflet contact member comprising a frame member having a curved central portion between a first side portion and a second side portion, wherein said curved central portion is configured to extend into the ventricle upon implantation and is engageable with at least one of the first leaflet or the second leaflet during ventricular systole; and
   a brace member extending from said leaflet contact member, wherein said brace member is configured to extend into the atrium and contact patient tissue so as to prevent retrograde motion of the implant.
20. The valve implant of embodiment 19, wherein a compliant material is secured to the frame member forming a leaflet contact surface, said leaflet contact surface configured such that said leaflet contact surface is engageable with one or more valve leaflets.
21. The valve implant of any one of embodiments 1-15, 19, or 20 wherein the valve implant is configured such that the ratio of the drag force of blood on the implant to the anchoring force of the implant is less than 1.
22. The valve implant of embodiment 20, wherein the compliant material is selected from the group consisting of: a mesh, a film, a fabric, a weave, a knit, a matrix, or a non-woven material.
23. The valve implant of embodiment 20, wherein said compliant material comprises polyesters, polyethers, polyolefins, polyurethanes, fluoropolymers, polyamides, elastomers, metals, and/or biologics.
24. The valve implant of embodiment 19, wherein said frame member has a first state with a reduced profile for implantation, and a second expanded state with a larger width.
25. The valve implant of embodiment 19, wherein said brace member comprises a first loop extending from the first side portion of said frame member and a second loop extending from the second side portion.
26. The valve implant of embodiment 25, wherein said first loop and said second loop each have a width between 2mm and 30mm.
27. The valve implant of embodiment 25, wherein said first loop and said second loop each have a height between 2mm and 50mm.
28. The valve implant of embodiment 19, wherein the frame member comprises a shape memory material.
29. A method for treating a mal-functioning mitral valve, the mitral valve having at least an annulus, a first leaflet, and second leaflet, defining a coaptation region between an atrium and a ventricle, said method comprising;
   implanting a cardiac valve implant between the first leaflet and the second leaflet, the cardiac valve implant having a leaflet contact member comprising a frame member having a curved central portion between a first side portion and a second side portion, wherein said curved central portion is configured to extend into the ventricle upon implantation and is engageable with at least one of the first leaflet or the second leaflet during ventricular systole; and a brace member attached to said leaflet contact member, wherein said brace member is configured to extend into the atrium and contact patient tissue so as to secure said leaflet contact member within the valve.
30. The method of embodiment 29, further comprising the step of providing a cardiac valve implant within a cannulated delivery device, the cardiac valve implant being expandable from a first compressed state to a second deployed state.
31. The method of embodiment 30, further comprising the step of introducing the cannulated delivery device into the atrium of the patient.
32. A method of forming a cardiac valve implant, the method comprising:
   attaching a compliant material to a frame member, wherein the frame member comprises a generally linear shape-memory material and wherein attachment of the compliant material causes the formation of a curve within the frame member.
33. The method of embodiment 32, wherein the frame member comprises a wire.
34. The method of embodiment 33, wherein the wire has a diameter of between 0.1mm to 1mm or between 0.1mm to 1.3mm.
35. The valve implant of any one of embodiments 1-15, or 19-28 wherein said frame member comprises a wire.
36. The valve implant of embodiment 35 wherein said wire has a diameter of between or between 0.1mm to 1mm or between 0.1mm to 1.3mm.
37. The valve implant of embodiment 36 wherein said wire material comprises nitinol.

## Claims

1. A cardiac valve implant for preventing regurgitation in a mitral valve, the mitral valve having a fibrous annulus and leaflets including a first leaflet, and a second leaflet, the leaflets defining a coaptation region between an atrium and a ventricle, said cardiac valve implant comprising:
a frame member having a curved central portion between a first side portion, and a second side portion, wherein said first side portion and said second side portion are configured to extend into the atrium, and wherein said curved central portion is configured to extend into the ventricle.

2. The valve implant of claim 1, wherein said curved central portion is configured to be engageable with at least one of the first leaflet or the second leaflet during ventricular systole.

3. The valve implant of claim 1, wherein a compliant material is secured to the frame member forming a leaflet contact surface, said leaflet contact surface configured such that said leaflet contact surface is engageable with one or more valve leaflets, preferably wherein said compliant material is secured to said frame member at said curved central portion, preferably wherein said compliant material is blood permeable and/or has multiple layers.

4. The valve implant of claim 3, wherein said compliant material comprises a mesh, a film, a fabric, a weave, a knit, a matrix, or a non-woven material, preferably wherein said compliant material comprises polyesters, polyethers, polyolefins, polyurethanes, fluoropolymers, polyamides, elastomers, metals, and/or biologics.

5. The valve implant of any one of claims 1-4, wherein said frame member comprises a shape memory alloy, and said valve implant has a first state with a reduced profile for implantation, and a second deployed state with a larger width.

6. The valve implant of claim 1, wherein said first side portion comprises a first attachment region securable to patient tissue, and wherein said second side portion comprises a second attachment region securable to patient tissue, preferably wherein said first attachment region comprises a first loop and said second attachment region comprises a second loop.

7. The valve implant of claim 6, wherein said first loop and said second loop each have a width between 2mm and 30mm and/or a height between 2mm and 50mm.

8. The valve implant of claim 6, wherein said first attachment region is configured to engage a first anchor member, and said second attachment region is configured to engage a second anchor member.

9. The valve implant of any one of claims 1-8 further comprising a brace member extending from said frame member, wherein said brace member is configured to extend into the atrium and contact patient tissue so as to prevent retrograde motion of the implant.

10. A cardiac valve implant for preventing leaflet regurgitation in a mitral valve, the mitral valve having a fibrous annulus and leaflets including a first leaflet, and a second leaflet, the leaflets defining a coaptation region between an atrium and a ventricle, said cardiac valve implant comprising:
a leaflet contact member comprising a frame member having a curved central portion between a first side portion and a second side portion, wherein said curved central portion is configured to extend into the ventricle upon implantation and is engageable with at least one of the first leaflet or the second leaflet during ventricular systole; and
a brace member extending from said leaflet contact member, wherein said brace member is configured to extend into the atrium and contact patient tissue so as to prevent retrograde motion of the implant.

11. The valve implant of any one of claims 1-10 wherein the valve implant is configured such that the ratio of the drag force of blood on the implant to the anchoring force of the implant is less than 1.

12. The valve implant of any one of claims 1-11 wherein said frame member comprises a wire, preferably wherein said wire has a diameter of between 0.1mm to 1mm or between 0.1mm to 1.3mm.

13. The valve implant of claim 12 wherein said wire material comprises nitinol.

14. A method of forming a cardiac valve implant, the method comprising:
attaching a compliant material to a frame member, wherein the frame member comprises a generally linear shape-memory material and wherein attachment of the compliant material causes the formation of a curve within the frame member.

15. The method of claim 14, wherein the frame member comprises a wire, preferably wherein the wire has a diameter of between 0.1mm to 1mm or between 0.1mm to 1.3mm.
